# EUROPEAN PATENT APPLICATION

(11) **EP 4 389 882 A1**
(43) Date of publication of application: **26.06.2024**
(21) Application number: 22215253.0
(22) Date of filing: 21.12.2022
(51) Int. Cl.: C12N 9/16, C12N 9/24, C12N 9/42, C12N 9/96, C12N 11/04, C12N 11/10

(54) **A METHOD FOR MANUFACTURING ENZYME GRANULES, AND ENZYME GRANULES**

(71) Applicant: AB Enzymes Oy, 05200 Rajamäki (FI)
(72) Inventor: PALMUNEN, Katja, 05200 Rajamäki (FI); LEHTIKARI, Leena, 05200 Rajamäki (FI); PERKKALAINEN, Mirkka, 05200 Rajamäki (FI)
(74) Representative: Espatent Oy

(57) **Abstract**

Herein is disclosed a method for manufacturing in a fluid bed granulator an enzyme granule comprising a core, an enzyme layer, and a coating, as well as a corresponding enzyme granule and a pellet containing the enzyme granule.

## Description

### FIELD OF THE INVENTION

The present invention relates to a method for manufacturing enzyme granules, to enzyme granules, and to pellets containing the enzyme granules.

### BACKGROUND OF THE INVENTION

Granulation of enzyme formulations is typically used to manufacture industrial enzyme products. Granulated enzymes can be used in pelleting to make feeds and in detergents.

### SUMMARY OF THE INVENTION

The inventors have found that stabile enzyme granules can be manufactured by a method described in the attached independent claims. The obtained enzyme granules maintain surprisingly well enzyme activity upon storage and in various applications, like in further processing of the granules into products such as to feed pellets. Similar advantages can also be achieved in detergents containing the present enzyme granules.

According to the first aspect of the invention is provided a method of manufacturing enzyme granules comprising:
i. feeding dry particle cores into a processing chamber of a fluid bed granulator;
ii. spraying into the processing chamber an enzyme formulation comprising an enzyme and a negatively charged macromolecule to obtain agglomerates; and
iii. coating the agglomerates with a coating agent to provide the enzyme granules.

In an embodiment the enzyme formulation further comprises:
at least one acid selected from acetic acid, formic acid, hydrochloric acid, phosphoric acid, and sulfuric acid; and/or
at least one base selected from sodium hydroxide and potassium hydroxide.

In an embodiment the enzyme formulation further comprises at least one carbohydrate. This embodiment is advantageous when using a core which does not contain carbohydrate.

In an embodiment the core, or the dry particle core, is selected from at least one of wheat flour, Na₂SO₄, maltodextrin, and starch. Preferably the dry particle core is a particle made of the above matter.

In an embodiment the core, or the dry particle core, does not contain carbohydrate and the enzyme formulation comprises carbohydrate. In another embodiment the dry particle core is a Na₂SO₄ particle and the enzyme formulation comprises carbohydrate. These embodiments are advantageous because they allow using dry particle cores without carbohydrate while providing sufficient amount of carbohydrate in the final enzyme granule product.

In an embodiment the negatively charged macromolecule is a polyanion.

In an embodiment an amount of the negatively charged macromolecule in the enzyme granule ((w/w) dry basis) is selected from the range 0.01 -10%, preferably 0.05 - 5%, more preferably 0.1 - 3%.

In an embodiment an amount of the enzyme in the enzyme granule ((w/w) dry basis) is selected from 1-80%, preferably 2-50%, more preferably 3-25%.

In an embodiment an amount of the carbohydrate amount in the enzyme granule ((w/w) dry basis) is selected from 10-99%, preferably 45-95%, more preferably 70-90%. Carbohydrate present in the enzyme granule increases stability of the enzyme granule.

According to another aspect of the invention is provided an enzyme granule manufactured by the present method and comprising:
(a) a core;
(b) an enzyme layer comprising a complex of the enzyme with a negatively charged macromolecule; and
(c) a coating.

In an embodiment the core is a dry particle core. Inside the core some enzyme formulation and its constituents may be present as a result of the spraying step described in the present manufacturing method. Similarly, some coating agent of the coating may be present in the enzyme layer. The layered structure of the present enzyme granule may thus not in all cases be a layered structure of sharply separated layers, but a layered structure with zones of a core, an enzyme layer, and a coating.

In another embodiment the enzyme granule comprises a core zone comprising the dry particle core and the enzyme formulation, and a coating on the core zone.

Layered structure of the granule can be verified by SEM analysis. In an embodiment the layered structure obtained by the manufacturing method is typical for the enzyme granules on average, while for an individual granule or granules the layered structure may not be clearly visible for each granule.

In an embodiment the enzyme granules have a porosity of at least 0.05cm³/g, preferably at least 0.07cm³/g, more preferably in the range 0.09-0.11cm³/g.

Porosity of the enzyme granules can be determined by a method of ISO 15901-1:2016 - Evaluation of pore size distribution and porosity of solid materials by mercury porosimetry and gas adsorption _ Part 1: Mercury porosimetry.

In an embodiment the enzyme granule has particle size in the range 100-2000µm.

According to another aspect of the invention is provided a pellet formed from the present enzyme granules.

### DETAILED DESCRIPTION

The scope of protection sought for various embodiments of the invention is set out by the independent claims. Any example or embodiment not falling within the scope of the independent claims is presented herein as an additional example or an additional embodiment useful for understanding the claimed invention.

Enzymes are catalytic proteins.

Proteins are polypeptides.

Polypeptides are long chains of amino acids linked by amide bonds. In an embodiment peptides are molecules composed of up to 20 amino acids, and polypeptides are molecules composed of more than 20 amino acids.

The generally accepted IUPAC single letter abbreviations for amino acids and their side chains in polypeptides are used herein.

The articles "a" and "an", as well as "another", are meant to refer to one or to more than one, that is to one or at least one, including several, of the grammatical object of "a", "an" or "another".

In an embodiment the method is an industrial scale method which optionally excludes for example analytical methods for research purposes.

In an embodiment any of the present methods is carried out in the sequence specified in a claim, aspect or embodiment.

The word "comprise" and variations thereof such as "comprises" and "comprising" are meant inclusively and include additional possible components that are technically compatible as understood by a person skilled in the art. These terms also may in certain embodiments include their narrow meaning "consisting of".

Recitation of ranges of values herein are merely intended to serve as a way of referring individually to each separate value falling within the range, unless otherwise indicated. Each separate value is thus disclosed in the specification as if it were individually recited. In an embodiment, and as is understood by the skilled person in the context in which the expression is used, an open-ended range such as "less than 10" is to be construed as a range disclosing values below 10, but above 0. Such a range can be understood to include a lower limit of e.g. 0.0001, 0.001, 0.1, or 1 depending on the context. A lower limit of an open ended range can also be determined by the skilled person such that at least one technical effect is observable or measurable, thereby excluding insignificant trace amounts.

In an embodiment the enzyme granule has retained at least 45%, preferably at least 70% of the enzyme activity present in the enzyme formulation before granulation.

The terms core and dry particle core refer to particle cores that are fed to the granulator and that form the innermost structure of the enzyme granule.

In an embodiment the enzyme granule has a coating which comprises or is made of the coating agent.

In an embodiment the enzyme granule has an enzyme layer which comprises or is made of the enzyme formulation.

Enzyme activity of a phytase enzyme can be determined according to Example 1 of WO2020094922.

The present method produces enzyme granules in which the enzyme molecule is protected upon storage. Protective effect can be evaluated for example based on heat stability of the granule by analyzing enzyme activity recovery when the granule is used in a pelleting process. With the present method it is possible to achieve a stable granule having pelleting processing recovery of ≥50% as shown in the Examples below. The protective effect was seen for examples in a test treatment at 95°C for 30s.

In an embodiment the present granulation method yields porous granules having Vᵢₙₜᵣₐ porosity above 0.05cm³/g. The granules obtained with the present method also have good dispersibility property. The particle size of the granule can be selected from the range 100-2000µm.

An enzyme to be used in the present granulation method, and in the present enzyme granule, can be isolated from its original biological source, or it can be produced in a cell-free system or produced as a secreted or intracellular protein in its original host or in an expression host, such as in a heterologous expression host. Such expression hosts include, but are not limited to, filamentous fungi, yeasts, bacteria, plants and algae, for example *Trichoderma reesei, Aspergillus oryzae, Pichia pastoris, Bacillus subtilis* and *Escherichia coli;* and were reviewed by Fernández et al. in Advanced Technologies for Protein Complex Production and Characterization 2016 pp 15-24, and by Yin et al. in Journal of Biotechnology 2007 335-347.

An enzyme according to the invention - and a gene encoding the enzyme according to the invention - can be derived from polypeptides and nucleic acids found in nature, or from a synthetic polypeptide or synthetic nucleic acid with sequence information derived from nucleic acids or polypeptides found in nature. Such sequence information can be derived from more than one sequence found in nature, for example calculation of a common ancestor sequence, calculation of a synthetic sequence from an alignment of known homologous sequences, particularly calculation of the most frequent amino acid at each position and derivation of a consensus sequence.

Furthermore, an enzyme according to the invention - and a gene encoding the enzyme according to the invention - can contain one or more alterations without loss of function. A functional enzyme can be verified for example by an enzyme activity measurement. Examples of such alterations are insertions, deletions and/or substitutions, preferably conservative substitutions, relative to a polypeptide or nucleic acid found in nature. The experimental exchangeability of amino acids in proteins has been reviewed by Yampolsky and Stoltzfus in Genetics 2005 pp 1459-1472 and can be applied to the enzymes used in the present method. Amino acid alterations are designated by their single letters separated by a slash, e.g. E/D means E or D. A particular example for such conservative substitutions are exchanges between E and D, because E and D are both acidic amino acids that differ only in one methylene spacer in their side chains. Further examples of the conservative substitutions are substitutions within the group of basic amino acids (e.g. R/K/H), acidic amino acids and their amides (e.g. E/D/N/Q), aromatic amino acids (e.g. W/F/Y), hydrophobic amino acids (e.g. F/L/I/V/A), tiny amino acids (e.g. G/A/S), medium amino acids (e.g. T/S/A/V/M/C), charged amino acids (e.g. E/D/R/K/H) and other polar amino acids (e.g. S/T/N/Q/H). Beside substitutions by the twenty canonical amino acids, also other genetically encoded amino acids, for example selenocysteine and pyrrolysine, and so-called unnatural amino acids can be incorporated in proteins. Example of such unnatural amino acids are reviewed by Wang et al. in Chemistry & Biology 2009 pp 323-336. By stepwise solid-phase peptide synthesis any available amino acid can be incorporated in peptides, which can be chemically ligated to larger peptides and polypeptides, to produce proteins and enzymes.

Furthermore, an enzyme according to the invention can be a fusion polypeptide in which another polypeptide and/or oligopeptide is fused at the amino- and/or carboxyl-terminus to a polypeptide. Examples of such oligopeptides and polypeptides are, but are not limited to poly histidine tags, signal peptides, linkers, binding domains, antibodies, chaperones, fluorescent proteins and enzymes.

The enzyme concentration in enzyme granules according to the invention is selected from 1 to 80% by weight. Such percentage is meant as active enzyme protein weight per total weight of composition on dry matter basis. In another embodiment the enzyme concentration is from 2 to 50% by weight and more preferable 3 to 25% by weight. Such percentages are expressed on dry matter basis.

In an embodiment the enzyme formulation further comprises in addition to the enzyme, fermentation broth, preferably clarified spent fermentation broth. Typical constituents present in a fermentation broth are compatible with the present method.

In another embodiment the dry matter content of the enzyme formulation without solids is selected from the range 0.1-25% w/w, such as 0.1-25, 0.1-20, 0.1-15, 0.1-10, 1-25, 1-20, 1-15, 1-10, 2-25, 2-20, 2-15, 2-10, 3-25, 3-20, 3-15, 3-10, 3-9, 3-8, 4-25, 4-20, 4-15, 4-10, 4-9, 4-8, 5-25, 5-20, 5-15, 5-10, 5-9, or 5-8 % w/w. In an embodiment the dry matter content refers to the dry matter present in the enzyme formulation without calculating the effect of the added negatively charged macromolecule or the added carbohydrate.

In an embodiment the enzyme formulation comprises enzyme in an aqueous medium. In another embodiment the enzyme formulation comprises enzyme in suspension and/or in solution, preferably in an aqueous medium. In another embodiment the enzyme formulation comprises an enzyme suspension in an aqueous medium. In another embodiment the enzyme formulation comprises an enzyme solution in an aqueous medium.

In an embodiment the pH of the enzyme formulation is set to a desired value before adding the negatively charged macromolecule. In another embodiment the pH is set to the selected value after adding the negatively charged macromolecule.

In an embodiment the enzyme formulation comprises carbohydrate. By including carbohydrates in the enzyme formulation granules can be made that have improved pelleting recoveries. This embodiment is effective when using in the granulation method core particles that do not contain any carbohydrate, or when a higher amount of carbohydrate in the enzyme granule is desired than can be obtained by using a carbohydrate-containing core only.

In an embodiment no carbohydrate is added into the enzyme formulation. Such an embodiment is advantageous when the core contains a high amount of carbohydrates, such at least 65% w/w, at least 70% w/w, 65-90% w/w, 70-90% w/w, 75-90% w/w, 80-90% w/w, 65-80% w/w, 70-80% w/w, or 75-80% w/w, and when the enzyme formulation contains a negatively charged macromolecule, such as a polyanion.

In an embodiment the negatively charged macromolecule is a polyanion, preferably a polyanion salt, more preferably a Na salt of the polyanion. Alternatively, the polyanion is a salt with another monovalent, such as a K salt, or a combination of a Na salt and a K salt.

In an embodiment the negatively charged macromolecule present in the enzyme granule is a polyanion, preferably a polyanion salt of the polyanion, more preferably a Na salt of the polyanion, a K salt of the polyanion, or a combination of a Na salt and of the polyanion and a K salt of the polyanion.

In an embodiment the polyanion is a polyanionic salt or a hydrolysate thereof. In an embodiment the polyanion is Na alginate, Na polypectate, Na hyaluronate, Na phytate, polyphosphate, or a combination thereof, or a combination of the polyanionic salt with at least one polyanion.

In an embodiment the polyanion forms a complex with the enzyme. Such complexes do not occur in a natural environment and they make the polyanion-enzyme complex different when compared to enzymes found in nature. Complex-formation can typically be observed by at least partial precipitation.

In an embodiment the polyanion is alginic acid, pectic acid, hyaluronic acid, phytic acid, or a salt of such a polyanionic acid, or any combination thereof.

In an embodiment the pH of enzyme formulation is adjusted to a range which is at least 0.2 pH units off from the calculated or measured pl of the enzyme. This range is particularly advantageous because within said ranges a good pelleting stability can be achieved.

In an embodiment the conductivity of the enzyme formulation is selected from the range 0-6000 µS/cm, such as 0.01-6000 µS/cm or 0.1-6000 µS/cm.

In an embodiment the enzyme is a phytase, and the pH of the enzyme formulation is adjusted with acetic acid, phosphoric acid, formic acid, sulphuric acid, and/or HCl. The pH is preferably set to an acidic value, more preferably to a value selected between 3 and 5, such as to about 4. The pH can be adjusted by changing the pH of the enzyme formulation which is sprayed during granulation. These embodiments are particularly useful to produce enzyme granules that are used in pelleting.

In an embodiment the enzyme is a phytase, and the negatively charged macromolecule is alginate, such as Na alginate. These embodiments are particularly useful to produce enzyme granules that are used in pelleting.

In an embodiment the enzyme is a phytase, and the pH of the enzyme formulation is set with NaOH to about 7. Preferably alginate, such as Na alginate, is used as the negatively charged macromolecule. These embodiments are particularly useful to produce enzyme granules that are used in pelleting.

In an embodiment the enzyme is a phytase. In a preferred embodiment the phytase is an *E*. *coli* phytase. In a preferred embodiment the phytase is an *Aspergillus* phytase. In a preferred embodiment the phytase is an *Buttiauxella* phytase. Preferably the phytase has phytate degrading activity. In an embodiment the phytase is as described in WO2020/094922 A1, Example 1.

In an embodiment at least one component of the enzyme formulation, preferably an enzyme formulation comprising phytase enzyme, has a different structural or physical characteristic compared to a corresponding natural component from which at least one component is derived from. In an embodiment the characteristic is uniform size, homogeneous dispersion in the composition, non-native glycosylation, non-native stability, production level, or purity. In an embodiment the phytase composition comprises a phytase-polyanion complex. Such a complex does not occur in a natural environment of phytases. In the present invention such a complex is however possible to achieve. Factors contributing to the complex formation are concentration of phytase and polyanion, pH, and ionic strength and temperature.

In an embodiment the enzyme, such as a phytase, is a bacterial enzyme, preferably a bacterial recombinant enzyme expressed in a heterologous host cell.

In an embodiment the enzyme formulation contains a 6-phytase having enzyme activity for 6-phos, preferably a protein engineered variant, chimeric or hybrid phytase. In another embodiment the enzyme formulation comprises phytases that are engineered to contain structural elements, such as domains, loops or binding sites, from two or more phytases. In an embodiment the phytase is a fungal phytase, preferably a fungal recombinant phytase expressed in a heterologous host cell.

In an embodiment the enzyme formulation contains a 3-phytase, preferably a protein engineered variant, chimeric or hybrid phytase. In another embodiment the hybrid phytase comprises phytases that are engineered to contain elements of two or more phytases.

In an embodiment the enzyme formulation contains a xylanase. In an embodiment a xylanase is an enzyme having xylan capability to degrade xylan. In an embodiment the xylanase has endo-β-1,4-xylanase activity (EC 3.2.1.8). In an embodiment the xylanase is as described in WO2005100557 A1, preferably as described in Example 5 ,6, 10, 12, or 15.

In an embodiment the enzyme formulation contains a cellulase. In an embodiment a cellulase is an enzyme having cellulolytic activity, which means that it is capable of hydrolysing cellulosic substrates or derivatives thereof into smaller saccharides. Cellulolytic enzymes thus include both cellulases and hemicellulases. Cellulases include (1) endoglucanases (EG, EC 3.2.1.4) which cut internal beta-1,4-glucosidic bonds; (2) exoglucanases or cellobiohydrolases (CBH, EC 3.2.1.176, EC 3.2.1.91) that cut the dissaccharide cellobiose from the reducing or non-reducing end of the crystalline cellulose polymer chain and (3) beta-1,4-glucosidases (BG, EC 3.2.1.21) which hydrolyse the cellobiose and other short cello-oligosaccharides to glucose. Cellulases can contain a cellulose binding module (CBM) that are separated from the catalytic domain by a flexible spacer known as a linker or linker peptide. In an embodiment the cellulase is as described in WO2020099719 A1, preferably as described in Example 1.

In an embodiment the complex is a reversible complex between the enzyme and the negatively charged macromolecule. In the reversible complex the enzyme and the negatively charged macromolecule join together to form a complex in which they interact and are bound directly to each other. Because the complex formation is reversible, the enzyme and the negatively charged macromolecule can be allowed to come apart for example by transferring the complex into aqueous solution, such as an activity analytic buffer solution used in Example 1 or 7.

In an embodiment the negatively charged macromolecule is a polyanion selected such that it is capable of forming a reversible complex, such as a complex between an enzyme molecule and a negatively charged macromolecule. One way of verifying complex formation is to analyse whether a precipitate forms between the enzyme and the polyanion when the polyanion is added to an enzyme formulation. When enzyme activity is retained when the precipitate is dissolved, the complex formation is considered to be reversible. Preferable examples of suitable negatively charged macromolecules are polyanions such as alginic acid, pectic acid, hyaluronic acid, and phytic acid or a thereof, or any combination thereof. These and other polyanions can be found an added for example from naturally occurring polysaccharides, gums or hydrogels like xanthan gum, gum arabic, heparin or carrageenan, or synthetic compounds having corresponding functions or characteristics.

In an embodiment the enzyme formulation comprises fermentation broth or clarified and optionally concentrated fermentation broth.

In an embodiment the coating comprises or is made of a coating agent.

In an embodiment the coating comprises a coating agent selected from at least one of: polyethylene glycol (PEG), PEG 4000, polyvinyl alcohol (PVA), hydroxypropylmethyl cellulose (HPMC), lecithin.

In an embodiment the coating agent is a water-soluble coating agent.

In an embodiment the coating agent is a non-water-soluble coating agent.

In an embodiment the coating agent is at least one of: polyethylene glycol (PEG), PEG 4000, polyvinyl alcohol (PVA), hydroxypropylmethyl cellulose (HPMC), lecithin.

The coating agent encapsulates the enzyme layer and the core and reduces dust formation.

In an embodiment the enzyme granule is dispersible to water.

When using the present granules to manufacture pellets, the stability of the enzyme is improved in the pellet, as shown in the examples by the increased pelleting processing recovery. The present granule thus stabilizes the enzyme and protects it from the high temperature and pressure that are used during pelleting.

In an embodiment the fluid bed granulator is a bottom spray granulator.

In an embodiment the granulator is operated at a temperature selected from the range 35-90°C, preferably from the range 50-80°C, more preferably from the range 60-65°C.

In an embodiment the fluid bed granulator is a bottom spray granulator equipped with separate spray nozzles connected to separate spray sources.

In an embodiment the fluid bed granulator is ProCell lab-unit granulator with spouted bed insert ProCell 5.

The present invention is further described by the following examples, which do not limit the scope of the invention, which is defined by the appended claims.

### EXAMPLES

### EXAMPLE 1. Dry enzyme granule showing importance of a soluble or a solid complex of an enzyme and a polyanion in the enzyme layer and importance of selecting the acid or base

Test formulations of dry granulated enzyme were produced using a fluid bed granulation process. The tests were carried out batch wise in Glatt ProCell lab-unit with spouted bed insert ProCell 5. With a bottom spray nozzle (two-substance nozzle) the spray liquid was atomized. Granulation temperature was 60 - 65°C. Enzyme used in all these granulations was phytase protein containing fermentation broth concentrate with 0.35% Na-benzoate as preservative. The phytase enzyme material and analytic methods were the same as described in WO2020094922 (A1), see in particular Example 1.

Dry granulated enzyme formulations shown in the tables below were used to study heat stability by standardized pelleting tests at an independent institute (Danish Technological Institute, Kolding, Denmark). Test formulations were mixed into mash feeds, which were treated in a conditioner with hot water steam resulting in different mash feed temperatures before pelleting. Recoveries were calculated from enzyme activities analysed in the pellets after heat treatment and compared to the enzyme activity in the mash feed without conditioning and pelleting.

These results show that heat stability in pellets after treatment at high conditioning temperatures is increased when using enzyme granules prepared from enzyme formulations comprising a soluble or a solid complex of an enzyme and a negatively charged macromolecule, such as a polyanion. Preferably a carbohydrate containing matrix is used as the spray liquid 1, i.e. as the enzyme formulation fed onto the fluid bed granulator. Pelleting was successful with or without at least one coating layer as an outer layer.

The table 1 shows the effect of a polyanion (here alginate or pectin) to achieve high pelleting recovery with enzyme granules based otherwise on equal amounts of maltodextrin, phytase and acetic acid with the same one-layer coating. Increase of recovery from 44% to 70 - 77% is seen when there is polyanion in the composition. Porosity of these granules is in the same range: 55 - 61 % where Vᵢₙₜᵣₐ 0.09 - 0.11 cm3/g analyzed by mercury intrusion porosimetry (MIP). Without polyanion and without coating the pelleting recovery is 40%.

Dispersibility of the granules was evaluated by simple test based on granule property forming lumps when dosed on water surface. In this test it was evaluated by visual assessment how easily the granules were disintegrated on contact with water in a beaker glass using 0.5 grams of granule sample and 100 ml of MQ-water at 25 ± 1 °C. The polyanion containing granules showed excellent dispersibility property.

**Table 1.**

| | Formulation | 1 | 2 | 3 | 4 |
|---|---|---|---|---|---|
| Layer | Component | % (w/w) dry basis | % (w/w) dry basis | % (w/w) dry basis | % (w/w) dry basis |
| Core material | | maltodextrin 18% | maltodextrin 18% | maltodextrin 19% | maltodextrin 19% |
| Spray 1 | carbohydrat e | maltodextrin 59% | maltodextrin 59% | maltodextrin 60% | maltodextrin 60% |
| | phytase | 15% | 15% | 15% | 15% |
| | polyanion | Na-alginate 1,5% | pectin 1,5% | - | - |
| | acid/base | acetic acid | acetic acid | acetic acid | acetic acid |
| | pH | 4 | 4 | 4 | 4 |
| Spray 2 | | HPMC 2% | HPMC 2% | HPMC 2% | - |
| Pelleting processi ng recovery (95°C, 30s) | | 77% | 70% | 44% | 40% |
| Porosity Vᵢₙₜᵣₐ (cm3/g) % | | 0.09 | 0.11 | 0.10 | NA |
| | | 61 | 57 | 55 | NA |
| Particle size | | | | | |
| x10-value (µm) | | 253 | 378 | 262 | 150 |
| x50-value (µm) | | 448 | 649 | 603 | 377 |
| x90-value (µm) | | 700 | 972 | 1152 | 686 |
| Dispersi bility | | Excellent | Excellent | Poor | Moderate |

The tables 2-4 show the effect of selecting the polyanion-acid/base combination to achieve good pelleting recovery. In each table there are comparable compositions to show effect of selected polyanion (here alginate or polyphosphate) combined with selected acid or base to adjust pH of spray 1 that creates the enzyme layer of the composition.

The tables 2-4 indicate that the used acid or base may have either beneficial or adverse effect in the composition matrix when evaluated based on the pelleting stability. They show that good recoveries ≥50% are possible to achieve in both cases, at pH 7 when phytase-alginate complex is mainly in soluble form and at pH 4 when it is mainly in solid form, when pH is adjusted with certain acid or base. Propionic acid or gluconic acid has adverse effect in the composition, while acetic acid, phosphoric acid, formic acid, HCl or NaOH has beneficial effect in the composition.

**Table 2.**

| | Formulation | 5 | 6 | 7 | 8 |
|---|---|---|---|---|---|
| Layer | Component | % (w/w) dry basis | % (w/w) dry basis | % (w/w) dry basis | % (w/w) dry basis |
| Core material | | maltodextrin 35% | maltodextrin 35% | maltodextrin 36% | maltodextrin 36% |
| Spray 1 | carbohydrate | maltodextrin 41% | maltodextrin 41% | maltodextrin 42% | maltodextrin 42% |
| | phytase | 17% | 17% | 16% | 16% |
| | polyanion | Na-alginate 1.5% | Na-alginate 1.5% | Na-alginate 1.5% | polyphosphate 1.5% |
| | acid/base | phosphoric acid | HCl | formic acid | acetic acid |
| | pH | 4 | 4 | 4 | 4 |
| Spray 2 | | HPMC 2% | HPMC 2% | HPMC 2% | HPMC 2% |
| Pelleting processi ng recovery (95°C, 30s) | | 89% | 62% | 70% | 50% |

**Table 3.**

| | Formulation | 9 | 10 |
|---|---|---|---|
| Layer | Component | % (w/w) dry basis | % (w/w) dry basis |
| Core material | | maltodextrin 8% | maltodextrin 7% |
| Spray 1 | carbohydrate | maltodextrin 68% | maltodextrin 71% |
| | phytase | 16% | 13% |
| | polyanion | Na-alginate 1.5% | Na-alginate 1.4% |
| | acid/base | NaOH | acetic acid |
| | pH | 7 | 4 |
| Spray 2 | | HPMC 2% | HPMC 2% |
| Pelleting processing recovery (95°C, 30s) | | 86% | 110% |

**Table 4.**

| | Formulation | 1 | 11 | 12 |
|---|---|---|---|---|
| Layer | Component | % (w/w) dry basis | % (w/w) dry basis | % (w/w) dry basis |
| Core material | | maltodextrin 18% | maltodextrin 18% | maltodextrin 18% |
| Spray 1 | carbohydrate | maltodextrin 59% | maltodextrin 58% | maltodextrin 58% |
| | phytase | 15% | 17% | 15% |
| | polyanion | Na-alginate 1.5% | Na-alginate 1.5% | Na-alginate 1.5% |
| | acid/base | acetic acid | propionic acid | gluconic acid |
| | pH | 4 | 4 | 4 |
| Spray 2 | | HPMC 2% | HPMC 2% | HPMC 2% |
| Pelleting processing recovery (95°C, 30s) | | 77% | 37% | 30% |

### EXAMPLE 2. Dry enzyme granules without and with different coatings

Dry granulated phytase compositions shown in the tables below were manufactured and studied as described in the example 1.

The Tables 5 and 6 show that good recoveries ≥70% are achieved with different coating strategies but also without coating with dry enzyme formulations based on maltodextrin, phytase, alginate and acetic acid.

**Table 5.**

| | Formulation | 13 | 14 |
|---|---|---|---|
| Layer | Component | % (w/w) dry basis | % (w/w) dry basis |
| Core material | | maltodextrin 8% | maltodextrin 7% |
| Spray 1 | carbohydrate | maltodextrin 63% | maltodextrin 70% |
| | phytase | 13% | 17% |
| | polyanion | Na-alginate 1.4% | Na-alginate 1.4% |
| | acid/base | acetic acid | acetic acid |
| | pH | 4 | 4 |
| Spray 2 | | lecithin 10% | HPMC 6% |
| Pelleting processing recovery (95°C, 30s) | | 107% | 84% |

**Table 6.**

| | Formulation | 15 | 16 | 17 |
|---|---|---|---|---|
| Layer | Component | % (w/w) dry basis | % (w/w) dry basis | % (w/w) dry basis |
| Core material | | maltodextrin 18% | maltodextrin 18% | maltodextrin 19% |
| Spray 1 | carbohydrate | maltodextrin 57% | maltodextrin 57% | maltodextrin 60% |
| | phytase | 15% | 15% | 15% |
| | polyanion | Na-alginate 1.6% | Na-alginate 1.5% | Na-alginate 1.5% |
| | acid/base | acetic acid | acetic acid | acetic acid |
| | pH | 4 | 4 | 4 |
| Spray 2 | | HPMC 4% | PVA 4% | - |
| Pelleting processin 9 recovery (95°C, 30s) | | 75% | 79% | 70% |

### EXAMPLE 3. Dry enzyme granules without carbohydrate in the enzyme layer

Dry granulated phytase compositions shown in the table below were manufactured and studied as described in the Examples 1-2.

The Table 7 shows importance of alginate with or without acetic acid in studied dry enzyme formulations having starch or maltodextrin as core material on pelleting recovery. It also shows that there is no need to have carbohydrate as part of the spray 1 like shown in the tables 1-6 if amount of the selected carbohydrate as a core material is high enough. However, it is preferable to use carbohydrate as part of the spray 1 to achieve higher pelleting recoveries shown in the previous tables 1-6. This example also shows that recovery over 50% is possible to achieve without any acid/base usage in the spray 1 but it is preferable to use the right polyanion-acid/base combination to achieve even higher recovery shown in the previous tables.

**Table 7.**

| | Formulation | 18 | 19 | 20 |
|---|---|---|---|---|
| Layer | Component | % (w/w) dry basis | % (w/w) dry basis | % (w/w) dry basis |
| Core material | | starch 71% | starch 72% | maltodextrin 68% |
| Spray 1 | carbohydrate | - | - | - |
| | phytase | 14% | 14% | 15% |
| | polyanion | Na-alginate 1.3% | - | Na-alginate 1.4% |
| | acid/base | acetic acid | - | - |
| | pH | 4 | 5 | 5 |
| Spray 2 | | PEG 4000 0,05% | PEG 4000 0,05% | PVA 9% |
| Pelleting processing recovery (95°C, 30s) | | 53% | 21% | 52% |

### EXAMPLE 4. Dry enzyme granules with different starting material / core

Dry granulated phytase compositions shown in the table below were manufactured and studied as described in the examples 1-3.

The Table 8 shows good pelleting recoveries of dry enzyme formulations prepared with various core materials.

**Table 8.**

| | Formulation | 21 | 22 |
|---|---|---|---|
| Layer | Component | % (w/w) dry basis | % (w/w) dry basis |
| Core material | | Na2SO4 19% | wheat flour 16% |
| Spray 1 | carbohydrate | maltodextrin 60% | maltodextrin 61% |
| | phytase | 15% | 16% |
| | polyanion | Na-alginate 1.6% | Na-alginate 1.6% |
| | acid/base | acetic acid | acetic acid |
| | pH | 4 | 4 |
| Spray 2 | | HPMC 2% | HPMC 2% |
| Pelleting processing recovery (95°C, 30s) | | 57% | 68% |

### EXAMPLE 5. Dry enzyme granules with different phytases

Dry granulated phytase compositions shown in the Table below were manufactured and studied as described in the Examples 1-4 except using different *E. coli* phytase molecule as the enzyme material in this example.

The Table 9 indicates robustness of dry enzyme formulations studied here with lower polyanion amount and different phytase molecule compared to the previous examples.

**Table 9.**

| | Formulation | 23 | 24 | 25 |
|---|---|---|---|---|
| Layer | Component | % (w/w) dry basis | % (w/w) dry basis | % (w/w) dry basis |
| Core material | | maltodextrin 8% | maltodextrin 14% | maltodextrin 43% |
| Spray 1 | carbohydrate | maltodextrin 68% | maltodextrin 63% | maltodextrin 34% |
| | phytase | 19% | 19% | 19% |
| | polyanion | Na-alginate 0.5% | Na-alginate 0.5% | Na-alginate 0.5% |
| | acid/base | phosphoric acid | sulphuric acid | phosphoric acid |
| | pH | 4 | 4 | 4 |
| Spray 2 | | HPMC 2% | HPMC 2% | HPMC 2% |
| Pelleting processing recovery (95°C, 30s) | | 91% | 81% | 80% |

### EXAMPLE 6. Dry enzyme granules with different enzymes

Dry granulated enzyme formulations were manufactured using cellulase and xylanase as the enzyme materials in this example.

Enzyme granules shown in the table below were manufactured as described in the examples 1-5. The granules were showing good enzyme stability profile evaluated based on over 50 % recovery either after pelleting processing (xylanase as disclosed in WO2005100557, see in particular Examples 5,6, 10, 12, 15) or after a detergent bleach test (cellulase as disclosed in WO2020099719, see in particular Example 1). The bleach test was performed as described in Example 7.

**Table 10.**

| | Formulation | 26 | 27 |
|---|---|---|---|
| Layer | Component | % (w/w) dry basis | % (w/w) dry basis |
| Core material | | maltodextrin 68% | wheat flour 45% |
| Spray 1 | carbohydrat e | maltodextrin 21% | maltodextrin 32% |
| | enzyme | cellulase 3% | xylanase 19% |
| | polyanion | Na-alginate 0.3% | Na-alginate 1.4% |
| | acid/base | acetic acid | NaOH |
| | pH | 4 | 7 |
| Spray 2 | | PVA 4% | HPMC 2% |

### EXAMPLE 7. Stability of cellulase granule in bleach detergent at extreme conditions

Cellulase granule having composition as shown in Example 6 was tested in the detergent bleach test as follows. Stability of cellulase granule was tested at extreme conditions by incubating the granules in outside laminated cardboard box containing bleach detergent at 32°C and 80% relative humidity for 3 weeks.

6 % of enzyme granule was mixed carefully with commercial bleach detergent powder in big plastic bottles. 150 g of enzyme-detergent mixture was packed in outside laminated cardboard box made in advance (size approx. 14.3 cm x 5.1 cm x 4.5 cm). First the bottom of boxes was glued with hotmelt glue, after filling the top was glued. Part of the mixture was saved for activity assay.

After incubating the enzyme-detergent mixture in climatic chamber Climacell (32°C, 80 % RH) for 3 weeks, the boxes were acclimatized for overnight at room temperature. The content of boxes was mixed well in big plastic bottle. Before analysing the enzyme activity, bleach inactivation was carried out as follows: 10 g of detergent-enzyme blend was mixed with 5 g Na2S2O3 (Merck 6512) and 250 ml HEPES buffer in a glass bottle and stirred for 20 minutes with magnetic stirrer. After that mixture was centrifuged and enzyme activity was measured as the release of reducing sugars from carboxymethylcellulose (3% CMC) at 50°C in 50 mM HEPES buffer, pH 7.0 essentially as described by Bailey, M. J. and Nevalainen, K.M.H., 1981; Haakana, H., et al, 2004 (NCU activity).

Results were calculated as residual activity (%) which was obtained by dividing the activity of sample after storage by the initial activity of the sample. After 3 week's storage at extreme condition (32°C, 80 % RH) the residual activity was still 55 %.

## Claims

1. A method of manufacturing enzyme granules comprising:
i. feeding dry particle cores into a processing chamber of a fluid bed granulator;
ii. spraying into the processing chamber an enzyme formulation comprising an enzyme and a negatively charged macromolecule to obtain agglomerates; and
iii. coating the agglomerates with a coating agent to provide the enzyme granules.

2. The method of claim 1, wherein the enzyme formulation further comprises:
at least one acid selected from acetic acid, formic acid, hydrochloric acid, phosphoric acid, and sulfuric acid; and/or
at least one base selected from sodium hydroxide and potassium hydroxide.

3. The method of any one of the claims 1-2, wherein the enzyme formulation further comprises at least one carbohydrate.

4. The method of any one of the claims 1-3, wherein the dry particle cores are selected from at least one of wheat flour, Na₂SO₄, maltodextrin, and starch.

5. The method of any one of the claims 1-4, wherein the negatively charged macromolecule is a polyanion.

6. The method of any one of the claims 1-5 wherein an amount of the negatively charged macromolecule in the enzyme granule ((w/w) dry basis) is selected from the range 0.01-10%, preferably 0.05-5%, more preferably 0.1-3%.

7. The method of any one of the claims 1-6, wherein an amount of the enzyme in the enzyme granule ((w/w) dry basis) is selected from 1-80%, preferably 2-50%, more preferably 3-25%.

8. The method of any one of the claims 1-7, wherein an amount of the carbohydrate in the enzyme granule ((w/w) dry basis) is selected from 10-99%, preferably 45-95%, more preferably 70-90%.

9. An enzyme granule manufactured by the method of any one of the claims 1-8 and comprising:
(a) a core;
(b) an enzyme layer comprising a complex of an enzyme with a negatively charged macromolecule; and
(c) a coating.

10. The enzyme granule of claim 9 having a porosity of at least 0.05cm³/g, preferably at least 0.07cm³/g, more preferably in the range 0.09-0.11cm³/g.

11. The enzyme granule of any one of the claims 9-10 having a particle size in the range 100-2000µm.

12. The enzyme granule of any one of the claims 9-11, wherein the complex is a reversible complex between the enzyme and the negatively charged macromolecule.

13. The enzyme granule of any one of the claims 9-12, wherein the negatively charged macromolecule is a polyanion, preferably a polyanion salt of the polyanion, more preferably a Na salt of the polyanion, a K salt of the polyanion, or a combination of a Na salt of the polyanion and a K salt of the polyanion.

14. The enzyme granule of any one of the claims 9-13, wherein the coating comprises a coating agent selected from at least one of: polyethylene glycol (PEG), PEG 4000, polyvinyl alcohol (PVA), hydroxypropylmethyl cellulose (HPMC), lecithin.

15. A pellet formed from the enzyme granules of any one of the claims 9-14.
